# EUROPÄISCHE PATENTSCHRIFT

(11) **EP 2 676 597 B1**
(45) Veröffentlichungstag und Bekanntmachung des Hinweises auf die Patenterteilung: **10.08.2016**
(21) Anmeldenummer: 13172929.5
(22) Anmeldetag: 20.06.2013
(51) Int. Cl.: A61B 1/00, A61B 1/018

(54) **Endoskophülle, Endoskopanordnung und Verfahren zum Bereitstellen einer Endoskopanordnung**
Endoscope sheath, endoscope system and method for providing an endoscope system
Enveloppe d'endoscope, agencement d'endoscope et procédé de mise à disposition d'un agencement d'endoscope

(30) Priorität: 20.06.2012 DE 102012105370
(43) Veröffentlichungstag der Anmeldung: 25.12.2013
(73) Patentinhaber: Karl Storz GmbH & Co. KG, 78532 Tuttlingen (DE)
(72) Erfinder: Wagner, Sebastian, 78532 Tuttlingen (DE); Dallemagne, Dr. Bernard, 4052 Beauafys (BE); Hyunsoo, Dr. Chung, Seoul (KR)

(56) Entgegenhaltungen:
- US-A- 5 386 817
- US-A1- 2005 004 434
- US-A1- 2006 052 662
- US-A1- 2008 269 562
- US-A1- 2012 095 291

## Beschreibung

Die vorliegende Erfindung betrifft eine Endoskophülle nach dem Oberbegriff von Anspruch 1 sowie eine Endoskopanordnung und ein Verfahren zum Bereitstellen einer Endoskopanordnung.

Endoskopische Operationstechniken haben sich für eine Vielzahl chirurgischer Eingriffe durchgesetzt. Ein wesentlicher Vorteil endoskopisch durchgeführter Eingriffe ist, dass die Notwendigkeit eines großen Einschnitts in der Haut und im darunterliegenden Gewebe zur Zugänglichmachung des Operationsgebiets entfällt, wodurch die Belastung des Patienten und die Dauer einer stationären Behandlung verringert werden können. Stattdessen werden bei endoskopischen Operationen Endoskope bzw. endoskopische Instrumente durch eine oder mehrere kürzere Inzisionen oder auch durch einen natürlichen Zugangsweg zum Operationsgebiet geführt, wo die zur Durchführung des Eingriffs notwendigen chirurgischen Manipulationen durchgeführt werden können. Insbesondere dann, wenn ein natürlicher Zugangsweg genutzt wird, um in einem Hohlorgan einen Eingriff vorzunehmen oder durch eine Inzision in der Wand des Hohlorgans beispielsweise einen Eingriff im Bauchraum vorzunehmen, ist es vorteilhaft, ein flexibles Endoskop gemeinsam mit zur Durchführung des Eingriffs benötigten endoskopischen Instrumenten durch den natürlichen Zugangsweg einführen zu können.

Es sind deshalb Endoskophüllen entwickelt worden, die es ermöglichen, ein flexibles Endoskop gemeinsam mit einem endoskopischen Arbeitsinstrument zu einem Operationsgebiet zu führen.

Aus US 2012/0095291 A1 ist eine Endoskophülle bekannt, die einen ersten Kanal zum Einführen eines Endoskops aufweist, wobei eine Wand, die den ersten Kanal umschließt, einen zweiten Kanal aufweist, in den ein Operationsinstrument eingeführt werden kann. Der zweite Kanal ist in Form einer Helix um den ersten Kanal gewunden, so dass eine Längsrichtung des zweiten Kanals von einer Längsrichtung des ersten Kanals abweicht. Hierdurch wird erreicht, dass sich ein über das distale Ende des zweiten Kanals hinaus geführtes endoskopisches Arbeitsinstrument schräg zur Richtung des distalen Endes eines in den ersten Kanal eingeführten Endoskops erstreckt.

Gemäß DE 600 28 014 T2 umschließt eine steuerbare Endoskopumhüllung ein Endoskop, wobei die Umhüllung außerdem zwei Lumen aufweist, die Kanäle für die Einführung von chirurgischen Instrumenten bereitstellen. Zur Steuerung der Ablenkung des distalen Endes der Lumen ist ein Drahtelement vorgesehen, das sich entlang der Wände der Lumen und angrenzend an das Endoskop erstreckt.

In US 2006/0052662 A1 ist ein Endoskop mit einem Hygieneschutz offenbart, der eine Hülle umfasst, die an ihrem distalen Ende geschlossen und an der Frontseite für optische Informationen durchlässig ist und die in Achsrichtung des Endoskops auf dieses aufgerollt werden kann. Das Endoskop enthält ferner einen oder mehrere Arbeitskanäle, die sich parallel zum Endoskop erstrecken und am distalen Ende der Hülle offen enden. Die Arbeitskanäle sind nur mit dem distalen Ende der Hülle verbunden und sind zwischen der Außenseite des Endoskops und der Innenseite der Hülle angeordnet.

In US 5,386,817 wird eine Schutzhülle für ein Endoskop beschrieben, die einen langerstreckten hohlen Schaft mit einer aus einem flexiblen Material bestehenden Wand umfasst. Das distale Ende umfasst eine Endkappe mit einem durchsichtigen Fenster für die Endoskopoptik eines flexiblen Endoskops sowie in der Seitenwand im wesentlichen parallel zu einer Endoskopkammer, in die das distale Ende des flexiblen Endoskops eingeführt werden kann, verlaufende Zugangskanäle.

Aus US 2008/0269562 A1 ist ein Endoskopsystem mit schwenkbaren Armen bekannt, das ein schlauchförmiges Element, einen an einem proximalen Ende des schlauchförmigen Elements angeordneten Griff und einen oder mehrere schwenkbare Arme umfasst, die lösbar mit einem distalen Ende des schlauchförmigen Elements verbunden sind. Das schlauchförmige Element verfügt über entlang seiner Längsachse verlaufende Kanäle, wovon mindestens einer ein optischer Kanal und mindestens ein anderer ein Beleuchtungskanal ist. Die schwenkbaren Arme umfassen Führungskanäle zur Aufnahme von chirurgischen Werkzeugen. Die Arme können aus einem transparenten Material aufgebaut sein.

In US 2005/0004434 A1 ist ein Endoskopschaft mit einem Arbeitskanal zur Einführung von Arbeitsinstrumenten und einer Anzahl von Versorgungs- und/oder Funktionskanälen offenbart. Die Versorgungs- bzw. Funktionskanäle sind in Spiralen, Schlangenlinien oder in Zick-Zack-Form um den oder entlang des Arbeitskanals angeordnet und sind in einer Hülle des Arbeitskanals eingebettet.

Der vorliegenden Erfindung liegt die Aufgabe zugrunde, eine Endoskophülle der eingangs erwähnten Art hinsichtlich ihrer Verwendbarkeit bei endoskopischen Eingriffen zu verbessern. Diese Aufgabe wird durch eine Endoskophülle gemäß Anspruch 1 sowie, ggf. in vorteilhafter Weiterbildung, durch eine Endoskophülle gemäß den weiteren auf eine Endoskophülle gerichteten Ansprüchen gelöst.

Ferner ist es Aufgabe der vorliegenden Erfindung, eine hinsichtlich ihrer Verwendbarkeit bei endoskopischen Eingriffen verbesserte Endoskopanordnung und ein Verfahren zum Bereitstellen einer entsprechenden Endoskopanordnung anzugeben. Diese Aufgabe wird durch eine Endoskopanordnung gemäß Anspruch 6 sowie durch ein Verfahren gemäß Anspruch 7 gelöst.

Eine erfindungsgemäße Endoskophülle umfasst einen lang erstreckten flexiblen Schlauch, der ein Hauptlumen zur Aufnahme zumindest eines Abschnitts eines Schafts eines flexiblen Endoskops umschließt und der zumindest abschnittsweise flexibel ausgebildet ist. Das Hauptlumen ist insbesondere als in einer Längsrichtung durchgehender Hohlraum bzw. Kanal ausgebildet, in den der Schaft des flexiblen Endoskops von einem proximalen (d.h. benutzernahen) Ende der Endoskophülle zumindest bis in die Nähe eines distalen (d.h. benutzerfernen) Endes der Endoskophülle eingeführt, insbesondere eingeschoben werden kann. Im eingeführten Zustand kann mit einer an der Spitze des Endoskops angeordneten Beobachtungsoptik eine vor dem distalen Ende der Endoskophülle angeordnete Szene beobachtet werden. Hierfür kann etwa ein Bild der Szene durch ein an der Spitze des Endoskops angeordnetes Endoskopobjektiv erzeugt werden, das durch einen im distalen Endbereich des Endoskops befindlichen Bildaufnehmer aufgenommen und durch innerhalb des Schafts verlaufende Leitungen zum proximalen Endbereich des Endoskops weitergeleitet wird oder das durch ein innerhalb des Schafts verlaufendes geordnetes Bündel von Lichtleitfasern zur einer im proximalen Endbereich des Endoskops angeordneten Kamera weitergeleitet wird. Die endoskopische Szene, etwa eine Innenwand eines Hohlorgans, kann beispielsweise über ein innerhalb des Schafts angeordnetes Lichtleitsystem mit von einer proximal angeordneten Lichtquelle erzeugtem Beleuchtungslicht beleuchtbar sein. Der Schaft des Endoskops kann einen steuerbaren Abschnitt, insbesondere einen steuerbaren Endabschnitt, aufweisen, der aktiv um einen gewünschten Betrag in eine gewünschte Richtung abgewinkelt werden und hierfür vom proximalen Ende des Endoskops her gesteuert werden kann; hierfür können beispielsweise innerhalb des Schafts verlaufende Steuerungsdrähte vorgesehen sein. Nach dem Einführen zumindest eines Abschnitts des Schafts des flexiblen Endoskops verbleibt ein Kopf des flexiblen Endoskops, der beispielsweise eine Handhabe mit Bedienungselementen, Anschlüsse für Versorgungsleitungen und/oder eine endoskopische Kamera aufweisen kann, vorzugsweise im Bereich des proximalen Endes außerhalb der Endoskophülle.

Der lang erstreckte flexible Schlauch ist zumindest abschnittsweise aus einem flexiblen Material, beispielsweise aus Gummi oder einem relativ weichen Kunststoff, hergestellt. Zur Verbesserung der Flexibilität kann der Schlauch dünnwandig ausgeführt sein. Die Wandstärke kann im Bereich 0,3 bis 1,5 mm liegen. Der Schlauch kann beispielsweise durch ein Metallgeflecht oder eine Drahtröhre verstärkt sein. Die Endoskophülle kann insbesondere als Einwegprodukt zur einmaligen Benutzung vorgesehen sein. Vorzugsweise ist die Endoskophülle nicht aktiv steuerbar ausgebildet, sondern kann beispielsweise durch eine Steuerungseinrichtung eines in das Hauptlumen des Schlauchs eingeführten Endoskops gesteuert bzw. abgewinkelt werden.

Die Endoskophülle weist weiterhin mindestens einen Arbeitskanal zur Aufnahme zumindest eines Abschnitts eines flexiblen endoskopischen Arbeitsinstruments auf. Der Arbeitskanal ist als weiteres Lumen bzw. als vom proximalen zum distalen Endbereich des Schlauchs durchgehender Hohlraum ausgebildet. In der Regel ist der Arbeitskanal mit einem kleineren Durchmesser ausgebildet als das Hauptlumen, jedoch mit einem größeren Durchmesser als ein in einem in das Hauptlumen einführbaren flexiblen Endoskop vorhandener Endoskop-Arbeitskanal. Hierdurch kann die Verwendung von endoskopischen Instrumenten ermöglicht werden, deren Durchmesser zu groß ist, um durch einen solchen Endoskop-Arbeitskanal eingeführt zu werden. Das endoskopische Arbeitsinstrument weist an seinem distalen Ende ein Werkzeug auf, das über ein innerhalb eines Schafts des Arbeitsinstruments verlaufendes Übertragungsmittel vom proximalen Ende des Schafts her bedient werden kann. Der Schaft des Arbeitsinstruments ist zumindest abschnittsweise flexibel ausgebildet. Das endoskopische Arbeitsinstrument kann zumindest so weit in den Arbeitskanal einführt werden, dass mit dem Werkzeug die Ausführung von Manipulationen im Bereich des distalen Endes der Endoskophülle, insbesondere vor dem distalen Ende der Endoskophülle, möglich ist. Das Arbeitsinstrument kann einen steuerbaren distalen Endabschnitt aufweisen, der eine Abwinkelung des Werkzeugs zur Durchführung von Manipulationen in einer gewünschten Richtung und an einem gewünschten Ort innerhalb des Operationsgebiets ermöglicht. Vorzugsweise ist der Arbeitskanal derart ausgebildet, dass ein steuerbarer distaler Endabschnitt das Arbeitsinstrument über das distale Ende des Arbeitskanals hinaus geschoben werden kann. Proximalseitig angeordnete Bedienelemente zur Betätigung des Werkzeugs bzw. zur Steuerung des distalen Endabschnitts des Arbeitsinstruments können nach dem Einführen des endoskopischen Arbeitsinstruments in den Arbeitskanal an dessen proximalem Ende außerhalb der Endoskophülle verbleiben.

Erfindungsgemäß weist die Endoskophülle einen transparenten distalen Endabschnitt auf. Vorzugsweise nimmt der transparente distale Endabschnitt nur einen kleinen Teil der gesamten Länge der Endoskophülle ein und ist in vorteilhafter Weise steif ausgebildet. Insbesondere kann die Endoskophülle derart ausgebildet sein, dass ein flexibles Endoskop mit der Beobachtungsoptik des Endoskops bis in oder nahe an den transparenten distalen Endabschnitt, jedoch nicht bis zu dessen distalem Ende, das auch das distale Ende der Endoskophülle darstellt, geführt werden kann. Hierfür kann das Hauptlumen innerhalb des transparenten distalen Endabschnitts beispielsweise eine Stufe aufweisen, die ein weiteres Vorschieben des Endoskops verhindert, oder die Länge der Endoskophülle und die Länge des Endoskopschafts können entsprechend bemessen sein. Insbesondere bei einer Schräg- oder Seitblickoptik kann es aber auch vorgesehen sein, dass das flexible Endoskop bis zum distalen Ende der Endoskophülle geführt werden kann. Der transparente distale Endabschnitt der Endoskophülle ist insbesondere atraumatisch ausgeführt.

Dadurch, dass die Endoskophülle einen transparenten distalen Endabschnitt aufweist, wird es ermöglicht, beim Einführen der Endoskophülle durch einen Zugangsweg zu einem Operationsgebiet mit der Beobachtungsoptik eines in das Hauptlumen eingeführten flexiblen Endoskops einen distalen Randbereich der Endoskophülle zu beobachten und auf diese Weise die Einführung der Endoskophülle zu kontrollieren. So kann beispielsweise sicherer festgestellt werden, ob eine Krümmung der Endoskophülle einer Krümmung des Zugangswegs angepasst ist oder ob beispielsweise das weitere Einführen der Endoskophülle durch Gewebe behindert wird. Hierdurch kann die Einführung der mit einem flexiblen Endoskop und einem Arbeitsinstrument versehenen Endoskophülle durch einen Zugangsweg zu einem Operationsgebiet vereinfacht werden und Verletzungen können vermieden werden. Ferner kann dadurch, dass ein transparenter distaler Endabschnitt vorgesehen ist, die Ausleuchtung des Zugangswegs bzw. des körperinneren Hohlraums verbessert werden.

Gemäß einer bevorzugten Ausführungsform der Erfindung ist der transparente distale Endabschnitt vom Schlauch trennbar ausgebildet. Der distale Endabschnitt kann beispielsweise dadurch mit einem distalen Ende des Schlauchs lösbar verbindbar ausgebildet sein, dass dieser auf das distale Ende aufgeschoben, aufgeclipst oder auch beispielsweise aufgeschraubt werden kann. Auf diese Weise wird die Herstellung der Endoskophülle vereinfacht. Ferner wird es durch die trennbare Ausführung des distalen Endabschnitts ermöglicht, diesen während einer Operation auszutauschen, beispielsweise wenn dieser durch Kontakt mit einem Werkzeug des Arbeitsinstruments beschädigt worden ist. Erfindungsgemäß ist der Arbeitskanal in Form einer Schraubenlinie um das Hauptlumen geführt, wobei der Arbeitskanal vorzugsweise eine Steigung zwischen 5 und 50 cm gegenüber der Längsrichtung des Hauptlumens aufweist. Die Steigung kann am proximalen Ende größer als am distalen Ende sein. Beispielsweise kann sie proximal im Bereich 30 bis 50 cm und distal zwischen 5 und 10 cm betragen. Dadurch, dass der Arbeitskanal schraubenförmig um das Hauptlumen gewunden ist, kann es erreicht werden, dass die Endoskophülle in alle Richtungen gleichmäßig flexibel ist. Auch dann, wenn ein flexibles Endoskop in das Hauptlumen und ein endoskopisches Arbeitsinstrument in den Arbeitskanal eingeführt sind, kann auf diese Weise eine gleichmäßige Biegsamkeit in alle Richtungen erzielt werden, ohne dass eine Vorzugsrichtung besteht. Das in den Arbeitskanal eingeführte Arbeitsinstrument ist dann selbst schraubenförmig um das Endoskop gewunden; aufgrund der geringen Schrägstellung des Arbeitskanals relativ zum Hauptlumen wird hierdurch die Bedienbarkeit des Arbeitsinstruments nicht wesentlich eingeschränkt.

Erfindungsgemäß ist auch dann, wenn der Arbeitskanal in Form einer Helix um das Hauptlumen verläuft, der Arbeitskanal im Bereich des distalen Endes der Endoskophülle, nämlich innerhalb des transparenten distalen Endabschnitts, im Wesentlichen parallel zum Hauptlumen bzw. zur Fortsetzung des Hauptlumens im distalen Endabschnitt gerichtet. Hierdurch wird die Bedienbarkeit des Arbeitsinstruments, wenn dieses über das distale Ende der Endoskophülle hinaus geschoben ist, verbessert.

Erfindungsgemäß mündet das distale Ende des Arbeitskanals in einer bezüglich einer Längsachse des Hauptlumens schräg angeordneten Öffnung. Die Öffnung bildet somit eine Öffnungsebene, die nicht senkrecht zur Längsachse des Hauptlumens im distalen Endabschnitt der Endoskophülle angeordnet ist. Insbesondere ist die Öffnung gegenüber einer distalen Öffnung des Hauptlumens, die das distale Ende der Endoskophülle bildet, seitlich versetzt, wobei die Öffnungsebene nach außen gerichtet gekippt ist. Die Normale der Öffnungsebene liegt somit in einer Ebene, die durch die Mittellängsachsen des Hauptlumens und des Arbeitskanals in einem distalen Endabschnitt der Endoskophülle, insbesondere im transparenten distalen Endabschnitt, gebildet wird, schließt mit diesen jedoch einen Winkel ein, der beispielsweise größer als 45° sein kann. Hierdurch wird es nicht nur erleichtert, einen steuerbaren distalen Endabschnitt des Arbeitsinstruments von der Mittellängsachse des Hauptlumens fort gerichtet abzuwinkein, sondern es wird ferner eine Ausbildung des distalen Endabschnitts der Endoskophülle geschaffen, die atraumatischer ausgebildet ist und die Einführung durch einen Zugangsweg zum Operationsgebiet erleichtert und Verletzungen vermeiden hilft.

Erfindungsgemäß ist das distale Ende des Arbeitskanals derart ausgebildet, dass ein über das distale Ende des Arbeitskanals hinaus geschobenes Arbeitsinstrument gegenüber Mittellängsachse des Hauptlumens im distalen Endabschnitt nach außen abgewinkelt wird. Hierfür ist eine Nase an einer distalen Öffnung des Arbeitskanals angeordnet, durch die flexible endoskopische Arbeitsinstrument, wenn dieses über das distale Ende des Arbeitskanals hinaus geführt wird, nach außen gerichtet abgewinkelt wird. Insbesondere ist Nase auf der dem Hauptlumen zugewandten Seite des Arbeitskanals in der schräg angeordneten distalen Öffnung des Arbeitskanals auf dessen Innenseite angeordnet. Die Steigung und Höhe der Nase sind in vorteilhafter Weise derart gewählt, dass das Arbeitsinstrument mit einem solchen Winkel abgewinkelt wird, dass das Werkzeug innerhalb Sichtfelds der in das Hauptlumen eingeschobenen Endoskopoptik liegt. Auf diese Weise kann erreicht werden, dass endoskopische Manipulationen mit dem Werkzeug unter endoskopischer Sichtkontrolle ausgeführt werden können, dass das Werkzeug jedoch genügend weit außerhalb des Zentrums des Sichtfelds des Endoskops angeordnet ist, um die endoskopische Sicht auf die für den Eingriff relevanten Gewebestrukturen nicht zu behindern. Zusätzlich kann eine weitere Abwinkelung nach außen oder auch in Richtung zur Mittelängsachse des Hauptlumens durch eine Steuerung eines distalen Endabschnitts des Arbeitsinstruments möglich sein.

Gemäß einer bevorzugten Ausführungsform der Erfindung ist der Schlauch in einem proximalen Endbereich in mindestens zwei Teilschläuche geteilt, wovon der erste Teilschlauch einen proximalen Endbereich des Hauptlumens und der zweite Teilschlauch einen Abschnitt des Arbeitskanals umschließen. An seinem distalen Ende geht der mehrlumige Schlauch somit in mindestens zwei einlumige Schläuche über, die jeweils einen kreisförmigen Querschnitt aufweisen können. Auf diese Weise wird es ermöglicht, außerhalb Endoskophülle verbleibende Bedien- und Anschlusselemente des flexiblen Endoskops und des Arbeitsinstruments unabhängig voneinander zu bewegen und zu betätigen sowie ohne räumliche Einschränkungen an entsprechende Versorgungseinrichtungen anzuschließen.

Der erste und/oder der zweite Teilschlauch können eine Abdichtungsvorrichtung zur Abdichtung des Hauptlumens bzw. des Arbeitskanals aufweisen. Die Abdichtungsvorrichtung kann insbesondere ein Ventil umfassen, so dass auch dann, wenn beispielsweise während einer Operation das Arbeitsinstrument bzw. das Endoskop gewechselt werden muss, die Abdichtung erhalten bleibt und ein ggf. für die Durchführung des Eingriffs notwendiger Insufflationsdruck bzw. eine in das Operationsgebiet eingeleitete Spülflüssigkeit nicht durch das Hauptlumen bzw. durch den Arbeitskanal entweichen kann. Hierdurch wird die Durchführung endoskopischer Operationen weiter erleichtert.

Vorzugsweise weist die Endoskophülle eine Mehrzahl an Arbeitskanälen auf, die zumindest in ihrem distalen Endabschnitt entlang einem Umfang des Hauptlumens zueinander benachbart angeordnet sein können. Die mehreren Arbeitskanäle sind insbesondere auch im transparenten distalen Endabschnitt einander benachbart angeordnet. Dabei können die distalen Endöffnungen der Arbeitskanäle in einer gemeinsamen Öffnungsebene angeordnet sein oder jeweils eine eigene Öffnungsebene bilden. Die Arbeitskanäle können einander benachbart schraubenförmig um das Hauptlumen des Schlauchs herumgeführt sein. Dadurch, dass eine Mehrzahl von Arbeitskanälen vorhanden ist, wird es ermöglicht, eine Mehrzahl von Arbeitsinstrumenten einzuführen, wodurch die Endoskophülle für besonders vielfältige Operationen einsetzbar ist; dies gilt auch unabhängig vom kennzeichnenden Merkmal des Anspruchs 1.

Gemäß einer bevorzugten Ausführungsform der Erfindung ist eine Querschnittskontur des Schlauchs durchgehend konvex gekrümmt oder aber abschnittsweise gerade und in den übrigen Abschnitten konvex gekrümmt. Vorzugsweise ist der Schlauch in seinem gesamten Bereich, in dem dieser das Hauptlumen und den mindestens einen Arbeitskanal umschließt, mit einem derartigen Querschnitt ausgebildet. Der Schlauch weist somit zumindest in einem Teilabschnitt, vorzugsweise über seine gesamte in einen körperinneren Zugangsweg einzuführende Länge, umfangsweise keine konkav gekrümmten Bereiche auf. Hierdurch wird vermieden, dass sich in durch konkave Bereiche gebildeten Vertiefungen Fertigungsrückstände oder Körpersekrete ansammeln können, die bei einer Reinigung nur schwierig zu entfernen sind. Ferner wird durch die konvexe bzw. zumindest nicht konkave Krümmung der Oberfläche des Schlauchs eine optimale Ausnutzung eines endoskopischen Zugangswegs, etwa der Speiseröhre oder der Harnröhre, ermöglicht.

Gemäß einer nicht beanspruchten Ausführungsform weist der mindestens eine Arbeitskanal eine Innenauskleidung auf, die beispielsweise als flexibles Rohr und/oder als Beschichtung ausgebildet sein kann. Das flexible Rohr kann beispielsweise in einem steiferen und härteren Kunststoffmaterial als der Schlauch ausgeführt sein, kann durch einen schraubförmig gewundenen Metalldraht oder ein Metallgeflecht verstärkt sein oder kann als Wellschlauch ausgeführt sein, um die Kräfte, die bei der Betätigung des Arbeitsinstruments auftreten, aufnehmen zu können und um Beschädigungen des Arbeitskanals zu vermeiden. Die Beschichtung bzw. das Material des Rohrs kann insbesondere derart gewählt sein, dass die Reibung zwischen der Außenseite des Arbeitsinstruments und der Innenseite des Arbeitskanals verringert wird. Hierdurch wird die Bedienbarkeit des Arbeitsinstruments, das bei der Durchführung eines endoskopischen Eingriffs häufig gedreht und/oder hin und her geschoben werden muss, verbessert und insbesondere auch die Durchführung von Manipulationen mit Werkzeugen ermöglicht, zu deren Betätigung erhebliche Kräfte übertragen werden müssen.

Gemäß einer nicht beanspruchten Ausführungsform kann der Schlauch bzw. die Endoskophülle einstückig ausgebildet oder auch als Verbundaufbau hergestellt sein. Als Verbundaufbau kann beispielsweise eine Mehrzahl einlumiger Schläuche miteinander verbunden sein, beispielsweise miteinander verklebt oder verschweißt. Auch eine Innenauskleidung des Arbeitskanals kann mit dem Schlauch als Verbundaufbau ausgebildet sein. Ein derartiger Verbundaufbau kann beispielsweise durch Verbundextrusion aus unterschiedlichen Materialien hergestellt werden. Hierdurch wird eine besonders einfache Herstellung der Endoskophülle ermöglicht.

Gemäß einer nicht beanspruchten Ausführungsform weist die Endoskophülle, insbesondere der Schlauch, in einen proximalen und/oder distalen Endbereich Fixiermittel zum Fixieren der Endoskophülle bzw. des Schlauchs gegenüber einem in das Hauptlumen eingeführten flexiblen Endoskop auf. Die proximalen Fixiermittel können insbesondere einem Teilschlauch, in dem das Hauptlumen im proximalen Endbereich der Endoskophülle geführt ist, zugeordnet sein. Durch distal und/oder proximal angeordnete Fixiermittel kann erreicht werden, dass die Endoskophülle relativ zum Endoskopschaft in einer für die Einführung der Endoskophülle in den Zugangsweg und die Durchführung des endoskopischen Eingriffs geeigneten Position gehalten wird. So kann es beispielsweise vorgesehen sein, dass durch proximal oder distal angeordnete Fixiermittel das Endoskop bezüglich seiner Längsrichtung zum Schlauch fixiert wird. Insbesondere bei einem Schrägblickendoskop ist es jedoch auch vorteilhaft, wenn der Endoskopschaft rotatorisch relativ zur Endoskophülle fixiert wird. In besonders vorteilhafter Weise kann die Endoskophülle in ihrem proximalen und in ihrem distalen Endbereich jeweils durch Fixiermittel relativ zum Endoskopschaft rotatorisch festgelegt werden. Hierdurch kann es auch ermöglicht werden, eine Endoskophülle mit einem Schlauch mit parallel zueinander verlaufenden Hauptlumen und Arbeitskanal beim Zusammenbau mit einem flexiblen Endoskop derart zu verdrillen, dass der Arbeitskanal schraubenförmig um das Endoskop herum gewunden ist, und den Schlauch in dieser Position relativ zum Endoskop zu fixieren. Da der Schaft des flexiblen Endoskops relativ rotationssteif ist, kann hierdurch eine Anordnung mit schraubenförmig um das Endoskop gewundenem Arbeitsinstrument erreicht werden, bei der die Flexibilität im Wesentlichen in alle Richtungen dieselbe ist.

Die Erfindung betrifft ferner eine Endoskopanordnung, die eine wie oben beschriebene Endoskophülle, ein zum Einführen in das Hauptlumen der Endoskophülle geeignetes flexibles Endoskop und mindestens ein Arbeitsinstrument, das zum Einführen in den Arbeitskanal der Endoskophülle geeignet ist, umfasst.

Das flexible Endoskop umfasst einen zumindest abschnittsweise flexiblen, lang erstreckten Schaft, der insbesondere zur Einführung in einen körperinneren Hohlraum geeignet ist. Im distalen Endbereich des Endoskopschafts ist eine Beobachtungsoptik zur Beobachtung einer endoskopischen Szene in dem körperinneren Hohlraum angeordnet. Zur Aufnahme und Weiterleitung des endoskopischen Bildes vom distalen zum proximalen Endbereich des Endoskops kann beispielsweise ein innerhalb des Schafts verlaufendes geordnetes Bündel von Lichtleitfasern vorgesehen sein oder auch ein elektronischer Bildaufnehmer, etwa ein CCD-Chip, der im Bereich des distalen Endes des Schafts angeordnet ist, und dessen Signale über innerhalb des Schafts verlaufende elektrische Leitungen zum proximalen Endbereich übertragen werden. Ferner kann innerhalb des Schafts ein Lichtleitsystem angeordnet sein, um Licht an das distale Ende des Endoskops zu transportieren, wo es zur Beleuchtung des Hohlraums genutzt wird. Weiterhin kann der Endoskopschaft einen oder mehrere Endoskop-Arbeitskanäle zur Durchführung von endoskopischen Arbeitsinstrumenten vom proximalen zum distalen Endbereich des Schafts enthalten, mit deren Hilfe innerhalb des Hohlraums Manipulationen ausgeführt werden können. Die Endoskophülle kann insbesondere derart bemessen bzw. ausgebildet sein, dass das flexible Endoskop mit seiner Beobachtungsoptik bis in oder nahe an einen transparenten distalen Endabschnitt der Endoskophülle geführt werden kann, jedoch nicht bis zum distalen Ende der Endoskophülle. Hierdurch wird die Einführung der Endoskopanordnung erleichtert, insbesondere unter verbesserter endoskopischer Sicht ermöglicht.

Das Arbeitsinstrument umfasst einen flexiblen lang erstreckten Schaft, der ebenfalls zur Einführung in einen körperinneren Hohlraum geeignet ist. Das Arbeitsinstrument ist zur Durchführung von Manipulationen in dem Hohlraum ausgebildet und weist hierfür ein am distalen Ende des Schafts angeordnetes Werkzeug auf, beispielsweise ein mit zwei gegeneinander wirkenden Maulteilen ausgebildetes Fassinstrument oder eine HF-Elektrode. Das Werkzeug kann über ein innerhalb des Schafts verlaufendes Übertragungsmittel vom proximalen Ende des Schafts her bedient bzw. versorgt werden. Ein solches flexibles endoskopisches Arbeitsinstrument weist in der Regel keine eigene Optik zur Aufnahme eines endoskopischen Bildes auf. Der Durchmesser des mindestens einen Arbeitskanals ist insbesondere größer als ein Durchmesser eines Endoskop-Arbeitskanals des Endoskops, so dass das Arbeitsinstrument einen Schaft mit einem Durchmesser aufweisen kann, der zur Einführung in einen Arbeitskanal des Endoskops nicht geeignet ist.

Vorzugsweise ist das flexible Endoskop zumindest in einem Teilbereich steuerbar. Alternativ oder zusätzlich kann auch das Arbeitsinstrument zumindest in einem Teilbereich steuerbar ausgebildet sein. Wird der steuerbare Teilbereich des in die Endoskophülle eingeführten Endoskops bzw. des in die Endoskophülle eingeführten Arbeitsinstruments abgewinkelt, so wird auf diese Weise auch die Endoskophülle abgewinkelt. Ist somit das flexible Endoskop oder mindestens ein Arbeitsinstrument steuerbar, so ist die Endoskopanordnung, die die Endoskophülle, das in diese eingeführte flexible Endoskop und das in die Endoskophülle eingeführte mindestens eine Arbeitsinstrument umfasst, auf diese Weise steuerbar. Dadurch wird die Einführung der Endoskopanordnung durch einen, ggf. gekrümmten, Zugangsweg zu einem körperinneren Hohlraum erleichtert. Ferner kann die Endoskopoptik in dem Hohlraum auf einen gewünschten Teilbereich gerichtet bzw. ein Teilbereich des Hohlraums zur Durchführung der Manipulationen angesteuert werden. Insbesondere kann das mindestens eine Arbeitsinstrument einen steuerbaren Endabschnitt aufweisen, der über ein distales Ende des Arbeitskanals hinaus geführt werden kann, um in einer gewünschten Richtung und an einem gewünschten Ort innerhalb des Hohlraums unter endoskopischer Sicht die Manipulationen mit dem Werkzeug ausführen zu können.

Ein erfindungsgemäßes Verfahren zum Bereitstellen einer Endoskopanordnung umfasst die folgenden Schritte:
- Bereitstellen eines flexiblen Endoskops und eines flexiblen endoskopischen Arbeitsinstruments,
- Bereitstellen einer Endoskophülle, die einen lang erstreckten flexiblen Schlauch umfasst, der ein Hauptlumen zur Aufnahme zumindest eines Abschnitts eines Schafts des flexiblen Endoskops und mindestens einen parallel zum Hauptlumen verlaufenden Arbeitskanal zur Aufnahme zumindest eines Abschnitts des flexiblen endoskopischen Arbeitsinstruments umschließt, wobei die Endoskophülle vorzugsweise einen transparenten distalen Endabschnitt aufweist,
- Einführen zumindest eines Abschnitts des Schafts des flexiblen Endoskops in das Hauptlumen, so dass eine Beobachtungsoptik des Endoskops in einem distalen Endbereich der Endoskophülle angeordnet ist,
- Verdrehen des distalen Endbereichs der Endoskophülle relativ zu einem proximalen Endbereich um eine Mittellängsachse des Hauptlumens, so dass der Arbeitskanal schraubenförmig um die Mittellängsachse des Hauptlumens verläuft,
- drehfestes Fixieren der Endoskophülle in einem proximalen und einem distalen Endbereich relativ zum Endoskop, und
- Einführen zumindest eines Abschnitts des flexiblen endoskopischen Arbeitsinstruments in den Arbeitskanal.

Hierdurch kann eine Endoskopanordnung bereitgestellt werden, die auf einfache Weise durch einen Zugangsweg in einen körperinneren Hohlraum zur Durchführung von Manipulationen mit einem Werkzeug des Arbeitsinstruments einführbar ist. Ferner können hierbei endoskopische Arbeitsinstrumente eingesetzt und ggf. während des Eingriffs gewechselt werden, die aufgrund ihres Schaftdurchmessers nicht durch Arbeitskanäle des Endoskops eingeführt werden können. In vorteilhafter Weise ist das flexible Endoskop steuerbar, um zumindest einen distalen Endbereich der Endoskopanordnung zur Erleichterung der Einführung durch den Zugangsweg und zur Verbesserung der Erreichung eines Operationsfelds innerhalb des Hohlraums steuern zu können.

Der Schritt des Einführens des endoskopischen Arbeitsinstruments in den Arbeitskanal kann auch vor dem Verdrehen des distalen Endbereichs erfolgen; im ersteren Falle wird in vorteilhafter Weise das Arbeitsinstrument nur so weit eingeführt, dass ein Werkzeug des Arbeitsinstruments noch nicht über das distale Ende des Arbeitskanals hervorsteht. Vorzugsweise ist ein distaler Endabschnitt des Arbeitsinstruments steuerbar, um mit dem Werkzeug in einer gewünschten Richtung und an einer gewünschten Position Manipulationen ausführen zu können.

Es versteht sich, dass die vorstehend genannten und die nachstehend noch zu erläuternden Merkmale nicht nur in der jeweils angegebenen Kombination, sondern auch in anderen Kombinationen oder in Alleinstellung verwendbar sind, ohne den Rahmen der vorliegenden Erfindung zu verlassen.

Weitere Aspekte der Erfindung ergeben sich aus der nachfolgenden Beschreibung eines bevorzugten Ausführungsbeispiels und der beigefügten Zeichnung. Es zeigen:
Fig. 1 ein Ausführungsbeispiel einer erfindungsgemäßen Endoskophülle in teilweise aufgeschnittener perspektivischer Ansicht;
Fig. 2 einen Längsschnitt durch den distalen Endbereich der in Fig. 1 dargestellten Endoskophülle;
Fig. 3 den distalen Endbereich einer Endoskophülle gemäß einem weiteren Ausführungsbeispiel der Erfindung in teilweise aufgeschnittener perspektivischer Ansicht;
Fig. 4 den Schlauch einer Endoskophülle gemäß einem Ausführungsbeispiel der Erfindung in teilweise geschnittener Ansicht.

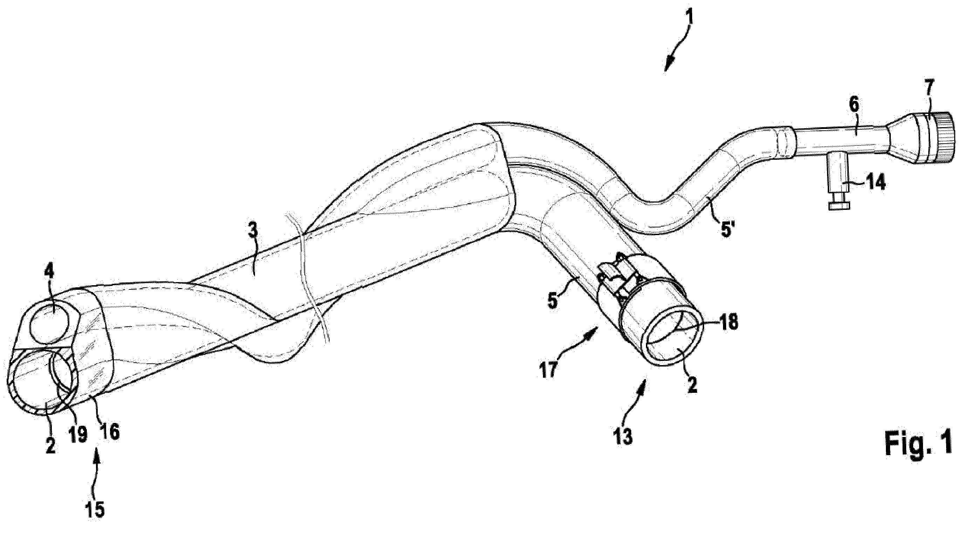

Wie in Fig. 1 beispielhaft dargestellt, umfasst eine erfindungsgemäße Endoskophülle 1 einen flexiblen Schlauch 3, der ein von einem proximalen Endbereich 13 zu einem distalen Endbereich 15 durchgehendes Hauptlumen 2 umschließt. Das Hauptlumen kann beispielsweise einen kreisförmigen Querschnitt mit einem Innendurchmesser von 12,5 mm aufweisen. Der flexible Schlauch 3 umschließt ferner einen Arbeitskanal 4, der ebenfalls vom proximalen Endbereich 13 zum distalen Endbereich 15 durchgehend ausgebildet ist und der beispielsweise im Querschnitt kreisförmig mit einem Innendurchmesser von 4,2 mm sein kann. Das Hauptlumen 2 ist zur Aufnahme des Schafts eines nicht dargestellten flexiblen Endoskops und der Arbeitskanal 4 zur Aufnahme des Schafts eines ebenfalls nicht dargestellten flexiblen endoskopischen Instruments ausgebildet. Im distalen Endbereich 15 ist auf den flexiblen Schlauch 3 eine steife, transparente Kappe 16 aufgesetzt, in der das Hauptlumen 2 und der Arbeitskanal 4 bis zum distalen Ende der Endoskophülle 1 weitergeführt sind und jeweils in distalen Endöffnungen münden. Die Kappe kann um beispielsweise 5 bis 25 mm über das distale Ende des Schlauchs 3 hinausragen.

Der Arbeitskanal 4 ist in Form einer Schraubenlinie (Helix) um das Hauptlumen 2 gewunden. Der Schlauch 3 ist nur in demjenigen Bereich seines Umfangs verdickt ausgebildet, in dem der Arbeitskanal 4 verläuft, so dass der Schlauch 3 mit einem nicht kreisförmigen, insbesondere länglichen Querschnitt ausgebildet ist, dessen Längsachse beim Fortschreiten entlang des Schlauchs 3 um das Hauptlumen 2 rotiert. In Fig. 1 ist der Querschnitt des Schlauchs 3 teilweise angedeutet, wobei in den übrigen Bereichen nur das Hauptlumen 2 und der Arbeitskanal 4 gezeigt sind und der beide umgebende Schlauch 3 nicht dargestellt ist. Der Winkel, um den der Arbeitskanal 4 gegenüber dem Hauptlumen 2 schräg verläuft, ist in Fig. 1 übertrieben dargestellt. Der elongierte Querschnitt der Kappe 16 ist ebenfalls in Fig. 1 erkennbar. Der Schlauch 3 kann aus einem Elastomer, aus PVC, PU, Silikon, PTFE, PP, PE oder einem ähnlichen Material hergestellt sein.,

Im proximalen Endbereich 13 teilt sich der Schlauch 3 in zwei Teilschläuche 5, 5', die das Hauptlumen 2 bzw. den Arbeitskanal 4 umschließen. Der Schlauch 3 kann mit den Teilschläuchen 5, 5' einstückig ausgebildet sein. Der Teilschlauch 5' ist mit einem starren Führungsrohr 6 verbunden, so dass der Arbeitskanal 4 durch den Teilschlauch 5' und das Führungsrohr 6 durchgehend bis zu einer am proximalen Ende des Führungsrohrs 6 angeordneten Dichteinheit 7 ausgebildet ist. Die Dichteinheit 7 kann insbesondere eine Dichtlippe und ein Ventil umfassen, durch das der Arbeitskanal 4 verschlossen ist, wenn kein Arbeitsinstrument in diesen eingeführt ist. Mit einer in Fig. 1 symbolisch dargestellten Befestigungsvorrichtung 14 kann das Führungsrohr 6 an einem nicht gezeigten Haltearm befestigt werden, um die Einführung und die Bedienung des Arbeitsinstruments zu erleichtern.

Der Teilschlauch 5, der einen proximalen Endabschnitt des Hauptlumens 2 umschließt, weist eine in Fig. 1 symbolisch als Spannring 17 dargestellte Fixiereinrichtung auf, durch die der Teilschlauch 5 und damit der Schlauch 3 auf einem in das Hauptlumen 2 eingeführten Schaft eines flexiblen Endoskops fixiert werden kann. Im distalen Endbereich 15 ist eine weitere Fixiervorrichtung angeordnet, beispielsweise eine Klemmlippe 19 aus Gummi, durch die der Schlauch 3 in seinem distalen Endbereich auf dem eingeführten Schaft des Endoskops fixiert wird. Dabei ist die Klemmlippe 19 derart bemessen, dass die gegenüber dem Endoskopschaft wirkende Haftreibung ausreicht, um eine axiale Verschiebung und eine Verdrehung des Schlauchs 3 gegenüber dem eingeführten Endoskopschaft bei der Benutzung in der Regel zu verhindern. Durch Anziehen des Spannrings 17 kann nach dem Einführen des Endoskopschafts ebenfalls sowohl eine axiale Verschiebung als auch eine Verdrehung des Schlauchs 3 gegenüber dem Endoskopschaft verhindert werden. Hierdurch wird es ermöglicht, eine in unverdrilltem Zustand, d.h. mit einem im Wesentlichen gestreckt und parallel zum Hauptlumen 2 verlaufenden Arbeitskanal 4, hergestellten Schlauch 3 durch Verdrillen des Schlauchs 3 die in Fig. 1 dargestellte Anordnung, bei der der Arbeitskanal 4 schraubenförmig um das Hauptlumen 2 verläuft, herzustellen; nachdem der Schaft des flexiblen Endoskops in das Hauptlumen 2 eingeführt ist, kann mit Hilfe der Klemmlippe 19 und des Spannrings 17 die verdrillte Anordnung fixiert und gegen Entdrillen gesichert werden. Hierdurch entsteht eine Anordnung mit einer Endoskophülle, die in alle Richtungen gleichmäßig flexibel ist und dadurch optimal an beliebig gekrümmte Zugangswege zu einem Operationsgebiet anpassbar ist.

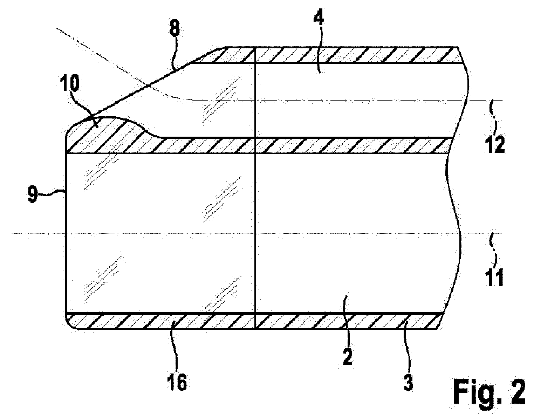

Wie in Fig. 1 angedeutet und in Fig. 2 im Querschnitt gezeigt, mündet der Arbeitskanal 4 an seinem distalen Ende in der Kappe 16 in einer schräg zur Längsachse des distalen Endabschnitts der Endoskophülle 1 angeordneten Endöffnung 8. Die Längsrichtung der Endoskophülle 1 im distalen Endabschnitt ist durch die Mittellängsachse 11 des Hauptlumens 2 gegeben, das in einer senkrecht zur Mittellängsachse 11 stehenden Endöffnung 9 mündet. Wie in Fig. 2 durch die Abwinkelung des distalen Bereichs der Instrumentenachse 12 eines in den Arbeitskanal 4 eingeführten flexiblen endoskopischen Instruments angedeutet ist, wird dieses beim Einführen über die Öffnung 8 hinaus durch die Nase 10, die als Verdickung der Zwischenwand zwischen dem Arbeitskanal 4 und dem Hauptlumen 2 ausgebildet ist, in einer von dem Hauptlumen 2 fort weisenden Richtung abgelenkt. Der Arbeitskanal 4 kann sich in der Kappe 16 in der durch die schraubenförmige Ausbildung gegebenen schrägen Richtung fortsetzen, ist jedoch bevorzugt in der Kappe 16 parallel zur Mittellängsachse 11 des Hauptlumens 2 geführt. Ein durch den Arbeitskanal 4 über die Öffnung 8 hinaus geschobenes Arbeitsinstrument ist somit in einer Richtung gerichtet, die mit der Mittellängsachse 11 des Hauptlumens einen Winkel bildet und dabei vorzugsweise in einer von der Mittellängsachse 11 und dem in der Kappe 16 befindlichen Teil der Instrumentenachse 12 gebildeten Ebene verläuft.

Wie in Fig. 2 angedeutet ist, sind die distalseitigen Kanten der Kappe 16 abgerundet, um das Einführen der Endoskophülle in einen körperinneren Hohlraum zu erleichtern und Verletzungen des Zugangswegs zu vermeiden. Aus dem gleichen Grund kann die Kappe 16 aus einem besonders weichen Material bestehen.

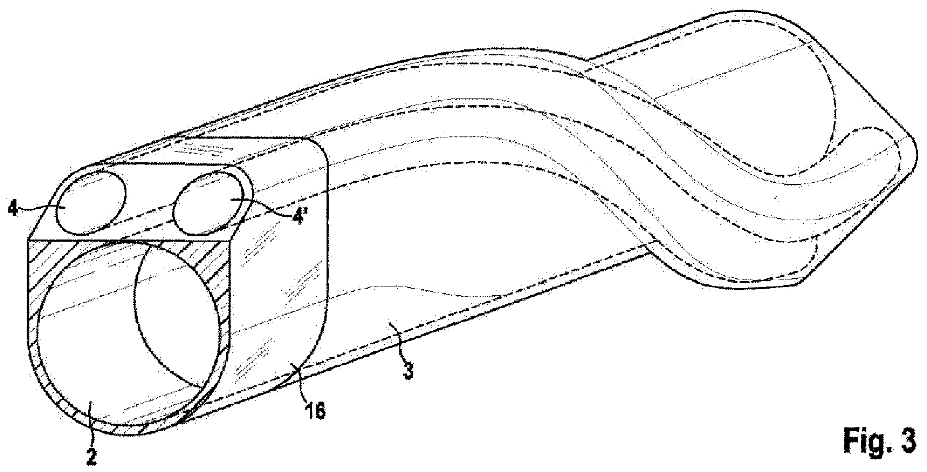

Gemäß einem weiteren Ausführungsbeispiel, dessen distaler Endbereich in Fig. 3 dargestellt ist, können der Schlauch 3 und die Kappe 16 mehrere Arbeitskanäle 4, 4' aufweisen, die insbesondere entlang einem Umfang des Hauptlumens 2 benachbart zueinander angeordnet sind und im Wesentlichen parallel zueinander und, ähnlich wie zu Fig. 1 beschrieben, schraubenförmig um das Hauptlumen 2 verlaufen. Hierdurch wird es ermöglicht, mehrere Arbeitsinstrumente, die miteinander zusammenwirken können, durch die Arbeitskanäle 4, 4' einzuführen und mit diesen unter endoskopischer Sicht Manipulationen durchzuführen. Im Übrigen ist das in Fig. 3 gezeigte Ausführungsbeispiel wie das in den Figuren 1 und 2 dargestellte ausgebildet, wobei eine derartige Endoskophülle in ihrem proximalen Endbereich für jeden der Arbeitskanäle 4, 4' einen eigenen Teilschlauch mit jeweils einem eigenen Führungsrohr und einer eigenen Dichteinheit aufweisen kann (nicht dargestellt).

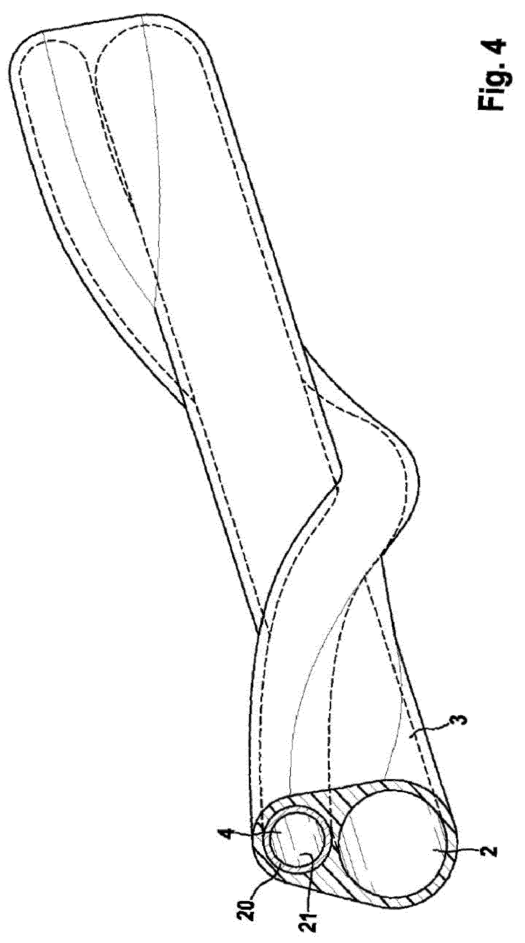

Wie in Fig. 4 gezeigt, kann der Arbeitskanal 4 zumindest innerhalb des Schlauchs 3 ein Innenrohr 20 aufweisen, das gegenüber dem Material des Schlauchs 3 steifer ausgebildet sein kann. Das Innenrohr kann beispielsweise durch einen schraubenförmig gewundenen Draht oder durch ein Drahtgeflecht verstärkt oder aus einem steiferen Kunststoff hergestellt sein. Hierdurch wird eine verbesserte Führung des Arbeitsinstruments auch unter Kraftausübung, wie diese bei der Durchführung der endoskopischen Manipulationen notwendig sein kann, ermöglicht. Auf der Innenseite des Innenrohrs kann eine reibungsvermindernde Beschichtung 21 angeordnet sein, um die Beweglichkeit des Arbeitsinstruments innerhalb des Arbeitskanals 4 zu verbessern. Die in Fig. 4 dargestellte Ausbildung des Arbeitskanals 4 kann bei dem in den Figuren 1 und gezeigten Ausführungsbeispiel verwirklicht sein, ebenso wie bei dem in Fig. 3 gezeigten Ausführungsbeispiel für die mehreren Arbeitskanäle 4, 4'.

Um eine für die Durchführung eines endoskopischen Eingriffs geeignete Endoskopanordnung zur Verfügung zu stellen, werden zunächst ein flexibles Endoskop mit einer für den geplanten endoskopischen Eingriff geeigneten Beleuchtungs- und Beobachtungsoptik sowie ggf. mit Saug- und Spülkanälen, ein für die Durchführung der beim Eingriff geplanten Manipulationen geeignetes flexibles endoskopisches Arbeitsinstrument und eine beispielsweise gemäß Fig. 1 ausgebildete Endoskophülle 1 mit einem Schlauch 3 bereitgestellt. Das Endoskop kann beispielsweise ein Standard-Gastroskop sein und das Arbeitsinstrument ein für den Einsatz in der Gastroskopie bestimmtes steuerbares endoskopisches Instrument. Die Endoskophülle 1 ist zur Aufnahme zumindest eines Abschnitts des Schafts des flexiblen Endoskops und zur Aufnahme zumindest eines Abschnitts des flexiblen endoskopischen Arbeitsinstruments bemessen. Der Arbeitskanal 4 der Endoskophülle 1 verläuft dabei im Wesentlichen parallel zu dem für die Aufnahme des Endoskopschafts vorgesehenen Hauptlumen 2.

Nunmehr wird ein Abschnitt des Schafts des Endoskops so weit in das Hauptlumen 2 des Schlauchs 3 der Endoskophülle 1 eingeschoben, dass die Beobachtungsoptik des Endoskops im Wesentlichen am distalen Ende des Schlauchs 3 angeordnet ist oder geringfügig über diese vorsteht. Auf das distale Ende des Schlauchs 3 wird eine steife, transparente Kappe 16 aufgesetzt. Die Beobachtungsoptik des Endoskops ist nun derart angeordnet, dass die Beobachtung einer vor dem distalen Ende der Endoskophülle 1, d.h. vor der Kappe 16, angeordneten endoskopischen Szene ermöglicht wird. Die Endoskopoptik kann hierfür beispielsweise als Geradeausblickoptik ausgebildet sein, wobei das über ein Endoskopobjektiv aufgenommene endoskopische Bild über einen Bildleiter zu einer im Handgriff des Endoskops angeordneten Kamera weitergeleitet wird. Das von der Kamera aufgenommene Bild kann auf einem Bildschirm für einen Benutzer sichtbar dargestellt werden.

Der distale Endbereich der Endoskophülle 1 bzw. des Schlauchs 3 wird nun relativ zu einem proximalen Endbereich des Schlauchs 3 in einem im Wesentlichen gestreckten Zustand des Schlauchs 3 um eine Mittellängsachse 11 des Hauptlumens 2 verdreht, so dass der Arbeitskanal 4 schraubenförmig um die Mittellängsachse 11 des Hauptlumens 2 verläuft. Der Schlauch 3 der Endoskophülle ist in seinem distalen Endbereich über die Klemmlippe 18 relativ zum Endoskop drehfest gehalten. Im proximalen Endbereich wird der Teilschlauch 5 des Schlauchs 3 mit Hilfe eines Spannrings 18 auf dem Schaft des flexiblen Endoskops geklemmt. Die schraubenförmig verdrehte Anordnung des Schlauchs 3 ist somit durch die Drehsteifigkeit des Endoskopschafts fixiert.

Ferner wird nun oder bereits in einem früheren Schritt das flexible endoskopische Arbeitsinstrument in den Arbeitskanal 4 eingeführt, der einen größeren Durchmesser aufweist als beispielsweise ein Arbeitskanal eines Gastroskops und dadurch die Verwendung von Arbeitsinstrumenten mit größerem Schaftdurchmesser zulässt. Dabei wird das Arbeitsinstrument zunächst nur so weit in die distale Richtung vorgeschoben, dass das Werkzeug noch nicht über die distale Endöffnung 8 in der Kappe 16 hinausragt. Nicht erfindungsgemäß ist es auch möglich, das Arbeitsinstrument erst nach Einführen der Endoskopanordnung in den körperinneren Hohlraum in den Arbeitskanal 4 einzuführen, oder dieses während des Eingriffs herauszuziehen und durch ein anderes zu ersetzen.

Um den geplanten Eingriff durchzuführen, wird die Endoskopanordnung aus der Endoskophülle und dem Endoskop sowie ggf. dem Arbeitsinstrument durch einen Zugangsweg in einen körperinneren Hohlraum eingeführt. Der Zugangsweg kann dabei beispielsweise die Speiseröhre sein, wenn innerhalb des Magens ein Eingriff, wie etwa eine endoskopische Probenentnahme, durchgeführt werden soll, oder auch wenn beispielsweise zur Durchführung einer Operation im Bauchraum ein Zugang durch das Hohlorgan eröffnet werden soll. Dabei kann die Endoskopanordnung durch eine Steuerungseinrichtung des Endoskops gesteuert werden und hierdurch die Einführung erleichtert und innerhalb des Hohlraums eine Ausrichtung des distalen Endes der Endoskopanordnung auf ein Operationsgebiet ermöglicht werden. Beim Einführung durch den Zugangsweg hält die Kappe 16 das Gewebe auf Abstand zum distalen Ende des Endoskops, so dass ein Rand der Kappe 16 beim Einführen sichtbar bleibt und eine sichere endoskopische Kontrolle des Einführvorgangs möglich ist. Auch das endoskopische Instrument ist innerhalb des in der Kappe 16 verlaufenden Teils des Arbeitskanals 4 sichtbar.

Das Arbeitsinstrument wird nach Erreichen des Operationsgebiets nun soweit vorgeschoben, dass das Werkzeug durch die Öffnung 8 über das distale Ende der Endoskophülle hinaus geschoben wird. Die Nase 10 bewirkt dabei eine Ablenkung des distalen Endabschnitts des Arbeitsinstruments, so dass dieses innerhalb des Sichtfelds der Beobachtungsoptik verbleibt, jedoch außerhalb der Bildmitte angeordnet ist. Durch Steuerung des distalen Endabschnitts des Arbeitsinstruments kann das Werkzeug bei unveränderter Position und Ausrichtung des Endoskops unter endoskopischer Kontrolle bewegt werden. Ferner kann das distale Ende der Endoskopanordnung mit dem Endoskop und dem Arbeitsinstrument durch Drehen des proximalen Endes des Endoskops gedreht werden. Hierdurch ist die Durchführung vielfältiger endoskopischer Eingriffe unter optimaler Kontrolle möglich.

Der Übersichtlichkeit halber sind nicht in allen Figuren alle Bezugszeichen dargestellt. Zu einer Figur nicht erläuterte Bezugszeichen haben die gleiche Bedeutung wie in den übrigen Figuren.

### Bezugszeichenliste

- 1: Endoskophülle
- 2: Hauptlumen
- 3: Schlauch
- 4,4': Arbeitskanal
- 5, 5': Teilschlauch
- 6: Führungsrohr
- 7: Dichteinheit
- 8: Endöffnung
- 9: Endöffnung
- 10: Nase
- 11: Mittellängsachse
- 12: Instrumentenachse
- 13: Proximaler Endbereich
- 14: Befestigungsvorrichtung
- 15: Distaler Endbereich
- 16: Kappe
- 17: Spannring
- 18: Dichtlippe
- 19: Klemmlippe
- 20: Innenrohr
- 21: Beschichtung

## Patentansprüche

1. Endoskophülle, umfassend einen lang erstreckten flexiblen Schlauch (3), der ein Hauptlumen (2) zur Aufnahme zumindest eines Abschnitts eines Schafts eines flexiblen Endoskops und mindestens einen als weiteres Lumen ausgebildeten Arbeitskanal (4, 4') zur Aufnahme zumindest eines Abschnitts eines flexiblen endoskopischen Arbeitsinstruments umschließt, wobei die Endoskophülle (1) einen transparenten distalen Endabschnitt (16) aufweist, **dadurch gekennzeichnet, dass** der Arbeitskanal (4, 4') in Form einer Schraubenlinie um das Hauptlumen (2) geführt ist und im distalen Endabschnitt parallel zu einer Mittellängsachse (11) des Hauptlumens (2) verläuft, wobei das distale Ende des Arbeitskanals (4, 4') in einer bezüglich einer Mittellängsachse (11) des Hauptlumens (2) schräg angeordneten Endöffnung (8) des distalen Endabschnitts (16) mündet, wobei das distale Ende des Arbeitskanals (4, 4') derart ausgebildet ist, dass ein über das distale Ende des Arbeitskanals (4, 4') hinaus eingeführtes Arbeitsinstrument gegenüber einer Mittellängsachse (11) des Hauptlumens (2) nach außen abgewinkelt wird, wobei in der Endöffnung (8) eine Nase (10) zur Abwinkelung des Arbeitsinstruments angeordnet ist.

2. Endoskophülle nach Anspruch 1, **dadurch gekennzeichnet, dass** der transparente distale Endabschnitt vom Schlauch (3) trennbar ausgebildet ist.

3. Endoskophülle nach einem der vorhergehenden Ansprüche, **dadurch gekennzeichnet, dass** der Schlauch (3) in seinem proximalen Endbereich (13) in mindestens zwei Teilschläuche (5, 5') geteilt ist, wovon einer das Hauptlumen (2) und ein anderer den Arbeitskanal (4) umschließt.

4. Endoskophülle nach einem der vorhergehenden Ansprüche, **dadurch gekennzeichnet, dass** eine Mehrzahl von Arbeitskanälen (4, 4') vorhanden ist, die zumindest in einem distalen Endabschnitt zueinander benachbart verlaufen.

5. Endoskophülle nach einem der vorhergehenden Ansprüche, **dadurch gekennzeichnet, dass** eine Querschnittskontur des Schlauchs (3) durchgehend konvex gekrümmt oder abschnittsweise gerade und in den übrigen Abschnitten konvex gekrümmt ist.

6. Endoskopanordnung umfassend eine Endoskophülle (1) nach einem der vorhergehenden Ansprüche, ein zur Einführung in das Hauptlumen (2) der Endoskophülle (1) bemessenes flexibles Endoskop und ein zur Einführung in den Arbeitskanal (4, 4') der Endoskophülle bemessenes flexibles endoskopisches Arbeitsinstrument.

7. Verfahren zum Bereitstellen einer Endoskopanordnung, wobei ein flexibles Endoskop, ein flexibles endoskopisches Arbeitsinstrument und eine Endoskophülle (1) nach einem der vorangegangenen Ansprüche bereitgestellt werden, wobei die Endoskophülle (1) einen lang erstreckten flexiblen Schlauch (3) umfasst, der ein Hauptlumen (2) zur Aufnahme zumindest eines Abschnitts eines Schafts des flexiblen Endoskops und mindestens einen parallel zum Hauptlumen verlaufenden, als weiteres Lumen ausgebildeten Arbeitskanal zur Aufnahme zumindest eines Abschnitts des flexiblen endoskopischen Arbeitsinstruments umschließt, und wobei die Endoskophülle (1) einen transparenten distalen Endabschnitt aufweist, wobei weiterhin zumindest ein Abschnitt des Schafts des flexiblen Endoskops in das Hauptlumen (2) eingeführt wird, so dass eine Beobachtungsoptik des Endoskops in einem distalen Endbereich (15) der Endoskophülle (1) angeordnet ist, der distale Endbereich (15) der Endoskophülle (1) relativ zu einem proximalen Endbereich (13) um eine Mittellängsachse (11) des Hauptlumens (2) verdreht wird, so dass der Arbeitskanal (4, 4') schraubenförmig um die Mittellängsachse (11) des Hauptlumens (2) verläuft, die Endoskophülle (1) in einem proximalen und einem distalen Endbereich (13, 15) relativ zum Endoskop drehfest fixiert wird, und wobei zumindest ein Abschnitt des flexiblen endoskopischen Arbeitsinstruments in den Arbeitskanal (4, 4') eingeführt wird, wobei alle Schritte vor einem Einführen der Endoskopanordnung in einen körperinneren Hohlraum erfolgen.

## Claims

1. Endoscope sheath, comprising an elongate flexible hose (3), which encloses a main lumen (2), for receiving at least one portion of a shaft of a flexible endoscope, and at least one working channel (4, 4'), designed as a further lumen, for receiving at least one portion of a flexible endoscopic working instrument, wherein (16), the endoscope sheath (1) has a transparent distal end portion **characterized in that** the working channel (4, 4') is guided in the form of a helical line around the main lumen (2) and, in the distal end portion, extends parallel to a central longitudinal axis (11) of the main lumen (2), wherein the distal end of the working channel (4, 4') opens out in an end opening (8) of the distal end portion (16) arranged obliquely with respect to a central longitudinal axis (11) of the main lumen (2), wherein the distal end of the working channel (4, 4') is designed in such a way that a working instrument inserted beyond the distal end of the working channel (4, 4') is angled outward in relation to a central longitudinal axis (11) of the main lumen (2), wherein a nose (10) for angling the working instrument is arranged in the end opening (8).

2. Endoscope sheath according to Claim 1, **characterized in that** the transparent distal end portion is designed to be separable from the hose (3).

3. Endoscope sheath according to one of the preceding claims, **characterized in that** the hose (3) is divided, in its proximal end area (13), into at least two sub-hoses (5, 5'), of which one encloses the main lumen (2) and another encloses the working channel (4).

4. Endoscope sheath according to one of the preceding claims, **characterized in that** a plurality of working channels (4, 4') are present which extend adjacent to one another at least in a distal end portion.

5. Endoscope sheath according to one of the preceding claims, **characterized in that** a cross-sectional contour of the hose (3) has a continuous convex curvature or is rectilinear in parts and has a convex curvature in the other parts.

6. Endoscope arrangement comprising an endoscope sheath (1) according to one of the preceding claims, a flexible endoscope dimensioned for insertion into the main lumen (2) of the endoscope sheath (1), and a flexible endoscopic working instrument dimensioned for insertion into the working channel (4, 4') of the endoscope sheath.

7. Method for providing an endoscope arrangement, wherein a flexible endoscope, a flexible endoscopic working instrument and an endoscope sheath (1) according to one of the preceding claims are provided, wherein the endoscope sheath (1) comprises an elongate flexible hose (3) which encloses a main lumen (2), for receiving at least one portion of a shaft of the flexible endoscope, and at least one working channel, designed as a further lumen, extending parallel to the main lumen and receiving at least one portion of the flexible endoscopic working instrument, and wherein the endoscope sheath (1) has a transparent distal end portion, wherein furthermore at least one portion of the shaft of the flexible endoscope is inserted into the main lumen (2) such that a viewing lens of the endoscope is arranged in a distal end area (15) of the endoscope sheath (1), the distal end area (15) of the endoscope sheath (1) is rotated relative to a proximal end area (13) about a central longitudinal axis (11) of the main lumen (2), such that the working channel (4, 4') extends helically around the central longitudinal axis (11) of the main lumen (2), the endoscope sheath (1) is fixed in rotation relative to the endoscope in a proximal and a distal end area (13, 15), and wherein at least one portion of the flexible endoscopic working instrument is inserted into the working channel (4, 4'), wherein all the steps are carried out before the endoscope arrangement is inserted into a cavity within the body.

## Revendications

1. Enveloppe d'endoscope, comprenant un tuyau flexible (3) s'étendant longitudinalement, qui entoure une lumière principale (2) pour recevoir au moins une portion d'une tige d'un endoscope flexible et au moins un canal de travail (4, 4') réalisé en tant que lumière supplémentaire pour recevoir au moins une portion d'un instrument de travail endoscopique flexible, l'enveloppe d'endoscope (1) présentant une portion d'extrémité distale transparente (16), **caractérisée en ce que** le canal de travail (4, 4') est guidé sous forme hélicoïdale autour de la lumière principale (2) et s'étend dans la portion d'extrémité distale parallèlement à un axe médian longitudinal (11) de la lumière principale (2), l'extrémité distale du canal de travail (4, 4') débouchant dans une ouverture d'extrémité (8) de la portion d'extrémité distale (16) disposée obliquement par rapport à un axe médian longitudinal (11) de la lumière principale (2), l'extrémité distale du canal de travail (4, 4') étant réalisée de telle sorte qu'un instrument de travail introduit au-delà de l'extrémité distale du canal de travail (4, 4') soit coudé vers l'extérieur par rapport à un axe médian longitudinal (11) de la lumière principale (2), un ergot (10) étant disposé dans l'ouverture d'extrémité (8) pour le coudage de l'instrument de travail.

2. Enveloppe d'endoscope selon la revendication 1, **caractérisée en ce que** la portion d'extrémité distale transparente est réalisée de manière à pouvoir être séparée du tuyau (3).

3. Enveloppe d'endoscope selon l'une quelconque des revendications précédentes, **caractérisée en ce que** le tuyau (3) est divisé dans sa région d'extrémité proximale (13) en au moins deux tuyaux partiels (5, 5') dont l'un entoure la lumière principale (2) et l'autre entoure le canal de travail (4).

4. Enveloppe d'endoscope selon l'une quelconque des revendications précédentes, **caractérisée en ce qu'**une pluralité de canaux de travail (4, 4') sont prévus, lesquels s'étendent les uns à côté des autres dans au moins une portion d'extrémité distale.

5. Enveloppe d'endoscope selon l'une quelconque des revendications précédentes, **caractérisée en ce qu'**un contour en section transversale du tuyau (3) présente une courbure convexe continue ou présente une portion droite et est pourvu d'une courbure convexe dans les autres portions.

6. Agencement d'endoscope comprenant une enveloppe d'endoscope (1) selon l'une quelconque des revendications précédentes, un endoscope flexible dimensionné pour être introduit dans la lumière principale (2) de l'enveloppe d'endoscope (1) et un instrument de travail endoscopique flexible dimensionné pour être introduit dans le canal de travail (4, 4') de l'enveloppe d'endoscope.

7. Procédé pour fournir un agencement d'endoscope, un endoscope flexible, un instrument de travail endoscopique flexible et une enveloppe d'endoscope (1) selon l'une quelconque des revendications précédentes étant fournis, l'enveloppe d'endoscope (1) comprenant un tuyau flexible (3) s'étendant longitudinalement, qui entoure une lumière principale (2) pour recevoir au moins une portion d'une tige de l'endoscope flexible et au moins un canal de travail réalisé en tant que lumière supplémentaire, s'étendant parallèlement à la lumière principale, pour recevoir au moins une portion de l'instrument de travail endoscopique flexible, et l'enveloppe d'endoscope (1) présentant une portion d'extrémité distale transparente, en outre au moins une portion de la tige de l'endoscope flexible étant introduite dans la lumière principale (2), de telle sorte qu'une optique d'observation de l'endoscope soit disposée dans une région d'extrémité distale (15) de l'enveloppe d'endoscope (1), la région d'extrémité distale (15) de l'enveloppe d'endoscope (1) étant tournée par rapport à une région d'extrémité proximale (13) autour d'un axe médian longitudinal (11) de la lumière principale (2), de telle sorte que le canal de travail (4, 4') s'étende sous forme hélicoïdale autour de l'axe médian longitudinal (11) de la lumière principale (2), l'enveloppe d'endoscope (1) étant fixée de manière solidaire en rotation par rapport à l'endoscope dans une région d'extrémité proximale et une région d'extrémité distale (13, 15) et au moins une portion de l'instrument de travail endoscopique flexible étant introduite dans le canal de travail (4, 4'), toutes les étapes s'effectuant avant l'introduction de l'agencement d'endoscope dans une cavité corporelle.
